# EUROPEAN PATENT APPLICATION

(11) **EP 4 837 591 A1**
(43) Date of publication of application: **11.11.2026**
(21) Application number: 25736189.9
(22) Date of filing: 02.01.2025
(51) Int. Cl.: A61K 47/69, A61K 47/62, A61K 47/54, A61K 47/24, A61K 47/28, A61K 33/42, B82Y 5/00, A61P 35/04, A61P 19/08

(54) **NANOMATERIAL FOR PREVENTING TUMOR BONE METASTASIS, PREPARATION METHOD THEREFOR, AND USE THEREOF**

(30) Priority: 03.01.2024 CN 202410007256; 21.02.2024 CN 202410194342
(71) Applicant: Zhejiang University, Hangzhou, Zhejiang 310000 (CN)
(72) Inventor: LIN, Xianfeng, Hangzhou, Zhejiang 310000 (CN); GU, Chenhui, Hangzhou, Zhejiang 310000 (CN); FAN, Shunwu, Hangzhou, Zhejiang 310000 (CN); TIAN, Hongsen, Hangzhou, Zhejiang 310000 (CN)
(74) Representative: Calysta NV
(86) International application number: PCT/CN2025/070168
(87) International publication number: WO 2025/146088

(57) **Abstract**

The present invention relates to a nanomaterial for preventing tumor bone metastasis, a preparation method therefore and use thereof. The present invention discovers a spatiotemporal coupling interaction between tumor cells and osteoclasts, and provides a tumor-bone initial metastasis behavior-targeting strategy, which may accurately prevent tumor metastasis based on the source, and may avoid drug resistance and biochemical resistance. Based on this, the present invention designs a nanomaterial for physical killing targeting tumor-osteoclast conjugates, and the nanomaterial is a bone-targeting group-modified nanovesicle encapsulating a carbonate compound and a phosphate compound. The nanomaterial can be effectively enriched in bone tissue; when the tumor cells are activated, an acid secretion function of tumor-related osteoclasts in the tumor-osteoclast conjugates in close contact triggers the carbonate compound to generate a carbon dioxide gas, and promotes the phosphate compound to release to form calcium phosphate crystals with a calcium ion, so as to kill nearby tumor cells, thereby achieving a specific very-early tumor metastasis inhibition effect.

## Description

### Cross-Reference to Related Application

This application claims priority to Chinese Patent Application No.202410007256.9 filed on January 3, 2024 and Chinese Patent Application No.202410194342.5 filed on February 21, 2024. Application Nos. 202410007256.9 and 202410194342.5 are hereby incorporated by reference in their entirety.

### Technical Field

The present invention relates to the technical field of tumor bone metastasis treatment, and specifically to a nanomaterial for preventing tumor bone metastasis, and a preparation method and use thereof.

### Background

Among many organs to which tumors metastasize, the bones are the most common primary site of metastasis for many malignant tumors, particularly breast cancer and prostate cancer. Meanwhile, the reprogramming of tumor cells in a bone microenvironment promotes secondary metastasis to other organs. Therefore, preventing bone metastases is a crucial step in preventing systemic tumor metastasis.

Unlike primary tumors, which are typically treated with local surgery or radiation therapy, tumor metastasis is a systemic, disseminated disease. Current treatment methods primarily include chemotherapy, targeted therapy, and immunotherapy, such as doxorubicin, bisphosphonates, PD-1 monoclonal antibodies, etc. However, the tumor cells are in a non-proliferative state during the long-term initial stage and evade immune surveillance by forming a tumor microenvironment. Due to the presence of isoforms, signaling bypasses, and tumor heterogeneity, therapies based on a single molecule or signaling pathway are prone to the development of drug resistance.

As the early tumor microenvironment is being established, inducing a conservative behavior in tumor stromal cells may itself represent a previously unrecognized new target for inhibiting a metastasis at the very earliest stage. Due to the unique "hard-base" interface of the bone matrix, "softening" the bone matrix is a prerequisite for initial metastasis. Therefore, osteoclasts, which are the only cells having acid secretion and osteolysis functions, play a key role in the initial microenvironment of tumor bone metastasis. The activation of the osteoclasts may clearly indicate the activation of bone metastasis lesions. Therefore, using the osteoclasts to trigger physical killing of the bone metastases is a promising method for avoiding drug resistance.

### Summary

The present invention reveals a spatiotemporal coupling interaction between tumor cells and osteoclasts, and provides a tumor-bone initial metastasis behavior-targeting strategy based on a spatiotemporal feature of tumor-osteoclast coupling during initial metastasis. An in-situ decoupling-killing liposome system triggered by the osteoclast induces physical killing via calcium phosphate crystals, such that tumor metastasis may be accurately prevented based on the source, and drug resistance and biochemical resistance may also be avoided.

In view of this, the present invention provides a nanomaterial for physical killing targeting a tumor-osteoclast conjugate, and a preparation method and use thereof. In order to implement the above objective, the present invention provides the following technical solutions.

In a first aspect, the present invention provides a nanomaterial. The nanomaterial is a bone-targeting group-modified nanovesicle encapsulating a carbonate compound and a phosphate compound.

In an implementation, the nanovesicle includes a nanoliposome.

In an implementation, the nanomaterial targets a tumor-osteoclast conjugate.

In an implementation, the nanomaterial can physically kill tumors. Preferably, physical killing is implemented by calcium phosphate crystals formed by the phosphate compound and a calcium ion.

In an implementation, an average particle size of the nanomaterial is 50-1000 nanometers; preferably, the average particle size is 50-200 nanometers; and most preferably, the average particle size is below 100 nanometers.

In an implementation, the bone-targeting group includes at least one of tetracycline, phosphonate, calcein, or an aspartate peptide sequence; the phosphonate, for example, is alendronate sodium; and preferably, the bone-targeting group is the tetracycline.

In the present invention, the carbonate compound can react with an acid to produce a gas; and preferably, the carbonate compound is selected from a carbonate salt or a bicarbonate salt, preferably selected from at least one of sodium bicarbonate, potassium bicarbonate, ammonium bicarbonate, sodium carbonate, potassium carbonate, or ammonium carbonate, and most preferably is the sodium bicarbonate.

In the present invention, the phosphate compound can form the calcium phosphate crystals with the calcium ion; and preferably, the phosphate compound is selected from a hydrogen phosphate salt or a dihydrogen phosphate salt, preferably selected from at least one of disodium hydrogen phosphate, dipotassium hydrogen phosphate, diammonium hydrogen phosphate, sodium dihydrogen phosphate, potassium dihydrogen phosphate, or an ammonium dihydrogen phosphate, and most preferably is the disodium hydrogen phosphate.

In an implementation, a molar ratio of the carbonate compound to the phosphate compound is 1-4:4-1, preferably 1-3:3-1 or 1-2:2-1, and more preferably 1:1.

In an implementation, a liposome membrane of the nanoliposome includes a bone-targeted phospholipid, phospholipids other than the bone-targeted phospholipid, and cholesterol.

Preferably, the bone-targeted phospholipid is obtained by covalently bonding a functionalized pegylated phospholipid and a bone-targeting group molecule. More preferably, the bone-targeted phospholipid is a bone-targeting group-pegylated phospholipid.

Preferably, the functionalized pegylated phospholipid refers to a pegylated phospholipid modified with a reactive functional group. The reactive functional group may be hydroxyl, carboxyl, amino, maleimide group, or the like. A molecular weight of polyethylene glycol may be 500-50000, preferably 800-6000.

Preferably, the functionalized pegylated phospholipid is functionalized DSPE-PEG; and more preferably, the functionalized pegylated phospholipid is DSPE-PEG-NHS.

Preferably, the functionalized pegylated phospholipid is DSPE-PEG2000-NHS or DSPE-PEG5000-NHS.

Therefore, preferably, the bone-targeted phospholipid is DSPE-PEG2000-tetracycline (DSPE-PEG2000-TC) or DSPE-PEG5000-tetracycline (DSPE-PEG5000-TC).

Preferably, the phospholipids other than the bone-targeted phospholipid are at least one of natural phospholipids, semi-synthetic phospholipids, or fully-synthetic phospholipids; preferably, the phospholipids other than the bone-targeted phospholipid are at least one of lecithin, hydrogenated lecithin, or cephalin; and more preferably, the phospholipids are lecithin.

Therefore, preferably, the liposome membrane includes the bone-targeted phospholipid, lecithin, and cholesterol.

Preferably, a mass ratio of the bone-targeted phospholipid, the phospholipids other than the bone-targeted phospholipid, and cholesterol is 5-40:100:10-25, preferably 15-25:100:12-20, and more preferably 20:100:16.

Preferably, a mass percentage of the bone-targeted phospholipid is 10-40 % of the liposome membrane, preferably 14-28%.

In a second aspect, the present invention provides a method for preparing a nanomaterial, including the following steps.

A bone-targeted phospholipid, phospholipids other than the bone-targeted phospholipid, and cholesterol are placed in an organic solvent, the solvent is removed, then a solution of a carbonate compound and a phosphate compound is added to perform ultrasonic hydration, the mixture is placed in a dialysis bag for dialysis, and extrusion granulation is performed to obtain a bone-targeting group-modified nanoliposome encapsulating the carbonate compound and the phosphate compound.

In an implementation, the organic solvent may be at least one of an alcohol solvent, an ester solvent, a halogenated hydrocarbon, a nitrile solvent, or an ether solvent; and preferably, the organic solvent may be at least one of methanol, ethanol, methyl acetate, ethyl acetate, dichloromethane, chloroform, acetonitrile, or ether.

In an implementation, a molar ratio of the carbonate compound to the phosphate compound is 1-4:4-1, preferably 1-3:3-1 or 1-2:2-1, and more preferably 1:1.

In an implementation, in the solution of the carbonate compound and the phosphate compound, a concentration of the carbonate compound is 30-120 mM, and a concentration of the phosphate compound is 20-80 mM; and more preferably, the concentration of the carbonate compound is 50-70 mM, and the concentration of the phosphate compound is 50-70 mM.

In an implementation, the bone-targeted phospholipid may be prepared by the following method: a functionalized pegylated phospholipid and a bone-targeting group molecule are covalently bonded, and the bone-targeted phospholipid is obtained through purification.

Preferably, reaction conditions for covalently bonding the functionalized pegylated phospholipid and the bone-targeting group molecule include: placing the functionalized pegylated phospholipid and the bone-targeting group molecule in an organic solvent, using an organic base to regulate the pH to 7.5-9.0, and performing, while stirring, a reaction for 6-48 hours at room temperature. The organic solvent may be at least one of an alcohol solvent, an ester solvent, a halogenated hydrocarbon, a nitrile solvent, or an ether solvent; and preferably, the organic solvent may be at least one of methanol, ethanol, methyl acetate, ethyl acetate, dichloromethane, chloroform, acetonitrile, or ether.

Preferably, after the stirring reaction, the solvent is replaced with water, and then the bone-targeted phospholipid is obtained through purification.

Preferably, a molar ratio of the functionalized pegylated phospholipid to the bone-targeting group molecule is 1:1-4, preferably 1:1-3, and more preferably 1:1-2.5.

Preferably, the pH is regulated to 8.0-8.4, and stirring is performed for 18-30 hours.

Preferably, purification is selected from at least one of dialysis or chromatography.

Preferably, through extrusion granulation, an average particle size of the nanoliposome obtained is 50-1000 nanometers; preferably, the average particle size is 50-200 nanometers; and most preferably, the average particle size is below 100 nanometers.

In a third aspect, the present invention further provides use of a nanomaterial. In the present invention, the nanomaterial is injected intravenously, and it is found that the nanomaterial may be enriched in bone tissue and released when a tumor is activated to induce a tumor-osteoclast conjugate, so as to form calcium phosphate crystals, which is used for physically killing the tumor-osteoclast conjugate in a bone metastasis lesion.

Therefore, the present invention provides use of a nanomaterial in preparation of drugs. The drugs are used for killing a tumor-osteoclast conjugate in a bone metastasis lesion. Furthermore, the present invention further provides a method for killing a tumor-osteoclast conjugate in a bone metastasis lesion. The method includes providing a nanomaterial to an individual in need.

In an implementation, the killing refers to physical killing. More preferably, the killing refers to physical killing caused by calcium phosphate crystals.

In an implementation, a bone metastasis lesion source includes at least one of breast tumor, prostate tumor, bone tumor (including osteosarcoma), lung tumor, liver tumor, kidney tumor, gastrointestinal tumor (including gastric tumor and intestinal tumor), or pancreatic tumor.

Furthermore, the present invention further provides use of a nanomaterial in preparation of drugs, and the drugs are for preventing tumor bone metastasis. Furthermore, the present invention further provides a method for preventing tumor bone metastasis. The method includes providing a nanomaterial to an individual in need.

In an implementation, tumors include at least one of breast tumor, prostate tumor, bone tumor (including osteosarcoma), lung tumor, liver tumor, kidney tumor, gastrointestinal tumor (including gastric tumor and intestinal tumor), or pancreatic tumor.

According to the nanomaterial of the present invention, when the tumor cells are activated, an acid secretion function of tumor-related osteoclasts in the tumor-osteoclast conjugates in close contact triggers the carbonate compound to generate a carbon dioxide gas, causing pores to form in the liposome membrane, and promotes the phosphate compound to release to form calcium phosphate crystals with a calcium ion, so as to kill nearby tumor cells, thereby achieving a specific very-early tumor metastasis inhibition effect. This physical killing method targeting a tumor cell behavior provides a new thought for targeting an early bone metastasis lesion and avoiding pharmacological tolerance and biochemical tolerance to tumors.

The present invention may also achieve similar technical effects by using other bone-targeting groups or other liposome component ratios.

Therefore, in a fourth aspect, the present invention further provides a method for preventing tumor bone metastasis. The method targets a tumor cell to induce an osteoclast behavior.

In an implementation, the method performs decoupling-killing on a tumor-osteoclast conjugate.

In an implementation, the method is triggered by a tumor-related osteoclast.

In an implementation, the method chemically or physically kills the tumor cell.

In an implementation, the method is performed via a bone-targeted drug.

Preferably, the bone-targeted drug is triggered by the tumor-related osteoclast.

Preferably, the bone-targeted drug has the capability of chemically or physically killing the tumor cell.

Preferably, the bone-targeted drug is the nanomaterial of the present invention.

In another aspect, the present invention studied the spatiotemporal features of tumor-induced osteoclast maturation. Since the development of classical osteoclasts involves continuous stimulation by M-CSF and RANKL, in order to explore a critical period for tumor-induced osteoclast formation, an osteoclast growth stage is divided into three key periods: an osteoclast precursor (OCP), an osteoclast precursor stimulated with RANKL for 1 day (R1-OCP), and an osteoclast precursor stimulated with RANKL for 5 days (R5-OCP). Through co-culture with the tumor cell, it is found that the R1-OCP can be induced to form an osteoclast. Through separated culture of the R1-OCP and the tumor cell using a Transwell chamber, it is found that the R1-OCP can only be induced to form the osteoclast under a spatial condition that the R1-OCP is in contact with the tumor cell.

Therefore, in a fifth aspect, the present invention further provides a method for culturing a tumor-related osteoclast. The method includes: an osteoclast precursor pre-stimulated by RANKL and a tumor cell are co-cultured in a contact manner, so as to obtain a tumor-related osteoclast.

Preferably, the osteoclast precursor pre-stimulated with the RANKL is an osteoclast precursor stimulated with the RANKL for 1-3 days, preferably the osteoclast precursor stimulated with the RANKL for 1 day.

Preferably, tumors include at least one of breast tumor, prostate tumor, bone tumor (including osteosarcoma), lung tumor, liver tumor, kidney tumor, gastrointestinal tumor (including gastric tumor and intestinal tumor), or pancreatic tumor.

In the present invention, the calcium phosphate crystals refer to crystals formed through the reaction of phosphate compounds with calcium ion, and have a main component being calcium carbonate.

Furthermore, the present invention further provides the following solutions.

The present invention provides a nanomaterial for physical killing targeting a tumor-osteoclast conjugate, and the nanomaterial is a bone-targeting group-modified nanoliposome encapsulating sodium bicarbonate and sodium hydrogen phosphate.

In an implementation, a particle size of the nanoliposome is 50-1000 nanometers; and preferably, the particle size of the nanoliposome is 100 nanometers.

In an implementation, the bone-targeting group includes tetracycline, phosphonate, or calcein.

The present invention provides a method for preparing a nanomaterial for physical killing targeting a tumor-osteoclast conjugate. The method includes the following steps.

S1, preparation of a bone-targeted phospholipid: a functionalized pegylated phospholipid and a bone-targeting group molecule are covalently bonded, and the bone-targeted phospholipid is obtained through dialysis and chromatography.

S2, preparation of a bone-targeting group-modified nanoliposome encapsulating sodium bicarbonate and sodium hydrogen phosphate: the bone-targeted phospholipid, lecithin, and cholesterol are dissolved in chloroform, and the mixture is rotary evaporated using a rotary evaporation; a mixed solution of the sodium bicarbonate and the sodium hydrogen phosphate is added to perform ultrasonic hydration, and extrusion granulation is performed to obtain the bone-targeting group-modified nanoliposome encapsulating the sodium bicarbonate and the sodium hydrogen phosphate.

In an implementation, the functionalized pegylated phospholipid is functionalized DSPE-PEG.

In an implementation, the functionalized pegylated phospholipid is DSPE-PEG2000-NHS to react with tetracycline; and reaction conditions include using triethylamine in the chloroform to regulate the pH to 8.0-8.4, and performing, while stirring, the reaction for 24 hours at room temperature.

In an implementation, a mass percentage of the bone-targeted phospholipid is 10-30% of a liposome membrane; the liposome membrane includes the bone-targeted phospholipid, lecithin, and cholesterol; and more preferably, a mass ratio of the bone-targeted phospholipid, lecithin, and cholesterol is 5:20:4.

In an implementation, a concentration of the sodium bicarbonate is 30-120 mM, and a concentration of the sodium hydrogen phosphate is 20-80 mM; and more preferably, the concentration of the sodium bicarbonate is 60 mM, and the concentration of the sodium hydrogen phosphate is 60 mM.

The present invention further provides use of a nanomaterial for physical killing targeting a tumor-osteoclast conjugate in physical killing of a tumor-osteoclast conjugate in a bone metastasis lesion.

In an implementation, a bone metastasis lesion source includes breast tumor, prostate tumor, bone tumor, lung tumor, liver tumor, kidney tumor, and gastrointestinal tumor.

The present invention has the following beneficial effects.
1) Triggered by the tumor-related osteoclast, the present invention performs "behavioral targeting" on the tumor cell, thereby avoiding the non-specific killing of conventional targeting methods.
2) The present invention physically kills the tumor cell by releasing a high concentration of hydrogen phosphate to rapidly form the calcium phosphate crystals with the in-situ calcium ion, thereby avoiding biochemical resistance in tumors.
3) The sodium bicarbonate and the disodium hydrogen phosphate used in the present invention are both physiologically environmental substances, which have high biosafety in drug metabolism.
4) The method for preventing a bone metastasis provided in the present invention targets the tumor cell to induce the behavior of the osteoclast. The method has a definite effect in the early prevention of the bone metastasis, provides more thoughts for the development of drugs to prevent the tumor bone metastasis, and thus is of broad significance.
5) The method for culturing an osteoclast in vitro provided in the present invention can obtain the tumor-induced osteoclast more effectively, providing a more powerful means for studying the spatiotemporal coupling between tumors and osteoclasts.

### Brief Description of the Drawings

In order to further clarify the objectives, technical solutions, and beneficial effects of the present invention, the following drawings are provided for illustrative purposes.
Fig. 1 is a fluorescence-TRAP diagram of a tumor-osteoclast conjugate in an in vivo bone metastasis lesion of a GFP-labeled tumor cell, where GFP fluorescence is used to position a tumor cell, and a TRAP signal is used to position an osteoclast.
Fig. 2 is a TRAP staining diagram of a tumor-osteoclast conjugate obtained from contact culture, where a difference in osteoclast induction displayed after OCP and R1-OCP are co-cultured with a 4T1 tumor cell indicates that RANKL pre-stimulation is a necessary time series condition for a tumor-related osteoclast.
Fig. 3 is a TRAP staining diagram of a tumor-osteoclast conjugate obtained from separated culture in Transwell, where the absence of an osteoclast after R1-OCP and a 4T1 tumor cell are cultured separately indicates that a close contact is a necessary spatial position condition for a tumor-related osteoclast.
Fig. 4 is a schematic diagram and a TRAP staining diagram of regional spatial culture, where R1-OCP that comes into contact only with a tumor cell is induced to form an osteoclast, and R1-OCPs outside the tumor cell are not induced to form the osteoclasts, verifying that a close contact with the tumor cell is a necessary spatial condition for a tumor-related osteoclast.
Fig. 5 is a schematic structural diagram of a tetracycline-modified sodium bicarbonate & disodium hydrogen phosphate nanoliposome (HC&HP@TNL)-a nanomaterial for physical killing targeting a tumor-osteoclast conjugate obtained according to Embodiment 2.1 of the present invention, where in a bar chart corresponding to each organ in the figure on the right, the left side represents ICG@NL, and the right side represents ICG@TNL.
Fig. 6 is a cryo-electron microscopy picture of calcium phosphate crystals formed before and after acid-responsive release of HC&HP@TNL obtained according to Embodiment 2.1 of the present invention (scale: 100 nanometers).
Fig. 7 is an element energy spectrum of calcium phosphate crystals formed after acid-responsive release of HC&HP@TNL obtained according to Embodiment 2.1 of the present invention.
Fig. 8 shows an in vivo imaging diagram and quantitative statistics of a bone-targeting ability of HC&HP@TNL obtained according to Embodiment 2.1 of the present invention.
Fig. 9 shows fluorescence intensity quantification of the release, at an interface between different cells and bone fragments, of HC&HP@TNL obtained in Embodiment 2.1 of the present invention.
Fig. 10 is a targeted release diagram of an in vivo bone metastasis lesion of HC&HP@TNL obtained according to Embodiment 2.1 of the present invention.
Fig. 11 is a TRAP staining diagram of the decoupling-killing capacity, for the coupling of tumor cells from different sources and a tumor-related osteoclast, of HC&HP@TNL obtained according to Embodiment 2.1 of the present invention.
Fig. 12 shows a tumor cell killing effect, which is characterized by a GFP expression signal, of HC&HP@TNL obtained according to Embodiment 2.1 of the present invention.
Fig. 13 is an electron microscope diagram of a tumor cell, physically killed by calcium phosphate crystals, of HC&HP@TNL obtained according to Embodiment 2.1 of the present invention.
Fig. 14 shows an inhibitory effect of HC&HP@TNL obtained according to Embodiment 2.1 of the present invention on tumor bone metastasis lesion in an in-vitro model compared with a classic anticancer drug DOX, where in Fig. d, bar charts in each group from left to right are Control, DOX, Cl@TNL, HC@TNL, and HC&HP@TNL in sequence.
Fig. 15 shows in vivo statistics of an inhibitory effect of HC&HP@TNL obtained according to Embodiment 2.1 of the present invention on a bone metastasis lesion.
Fig. 16 shows Micro-CT statistics of an inhibitory effect of HC&HP@TNL obtained according to Embodiment 2.1 of the present invention on bone loss at the metaphysis, where in each group of bar charts shown in the figure on the right, the left side represents Cl@TNL, and the right side represents HC&HP@TNL.

### Detailed Description of the Embodiments

A nanomaterial for physical killing targeting a tumor-osteoclast conjugate, and a preparation method and use thereof provided in the present invention are described below in detail with reference to embodiments. However, the embodiments should not be construed as limiting the scope of protection of the present invention.

The present invention is further described in detail below with reference to specific embodiments. It is also to be understood that, the following embodiments are only used to further illustrate the present invention and are not to be construed as limitations on the scope of protection of the present invention, and some non-essential improvements and adjustments made by those skilled in the art in accordance with the foregoing contents of the present invention are within the scope of protection of the present invention. The specific process parameters and the like of the following examples are also only an example of a suitable range, that is, those skilled in the art may select within a suitable range through the description herein, and is not necessarily limited to the specific numerical values of the following examples.

### Embodiment 1 Investigation of spatiotemporal coupling relationship between tumors and osteoclasts

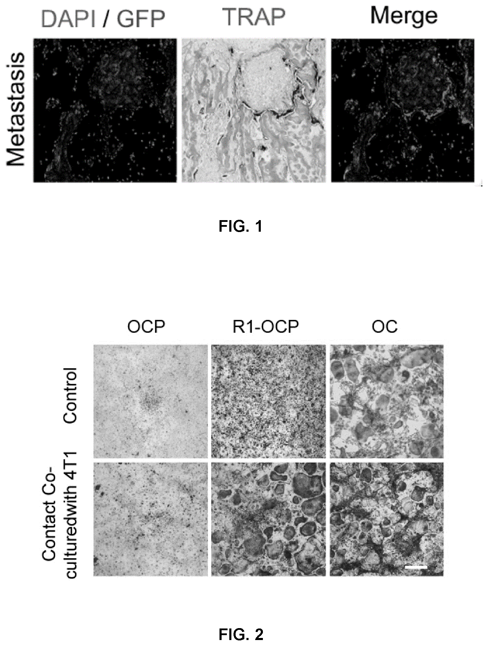

First, a relative relationship between a tumor metastasis lesion and an osteoclast was observed at an in vivo level in a mouse bone metastasis model, which was performed by the following steps.
(1) 8-to-10-week-old female Balb/c mice were selected and anesthetized.
(2) A 1.5-centimeter incision was formed in the lower right abdomen between the 4th and 5th nipples. The muscles were opened to expose the iliac artery.
(3) 0.1 mL of 5 × 10⁶/mL GFP-expressing 4T1 breast tumor cells were injected into the iliac artery using a 31G needle.
(4) The area of the arterial incision was pressed with a cotton-tipped swab to stop the bleeding, then the incision was sutured.
(5) 8 days later, consecutive tissue sections were performed from the right femur of the mice and subjected to fluorescence scanning and TRAP staining, respectively, to observe the positioning of a GFP-positive tumor cell and a TRAP-positive osteoclast, discovering significant colocalization and a "surrounding phenomenon" between a tumor-related osteoclast and a metastasis lesion (Fig. 1).

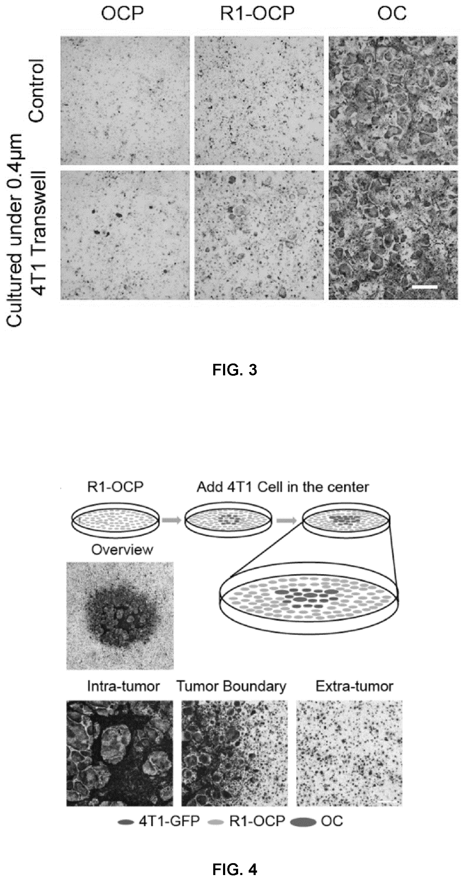

A cell culture model for investigating a spatiotemporal relationship between the tumor cell and the osteoclast was constructed, which was performed by the following steps.
(1) Mouse femurs were taken and rinsed with a culture medium to obtain bone marrow cells, which were then cultured for 5 days in an α-MEM culture medium containing 20 ng/mL M-CSF.
(2) The cells were digested and spread onto a plate, Day 0 was set, and the cells obtained at this stage were osteoclast precursors (OCP); 50 ng/mL RANKL was added to a culture system and cultured for 1 day; then the RANKL was removed; on Day 1, osteoclast precursors (R1-OCP) pre-stimulated with the RANKL for 1 day were obtained; and if the RANKL was not removed and the cells were continued to be cultured with the RANKL for 4 days, osteoclast precursors stimulated with the RANKL for 5 days (R5-OCP) were obtained on Day 5, which were also classic osteoclasts (OC) in a conventional culture system.
(3) On Day 1, 4T1 tumor cells were added to OCP, R1-OCP, and R5-OCP groups, respectively, and continuously cultured until Day 5. It was found that the 4T1 cells might induce the R1-OCP into osteoclasts, and the OCP did not have the ability to be induced by 4T1 cells into the osteoclasts (Fig. 2). Therefore, RANKL pre-stimulation was a necessary time series condition for a tumor-related osteoclast.
(4) Maintaining other conditions unchanged, the above 4T1 tumor cells were cultured on a Transwell chamber separated from the osteoclast precursors, it was found that the R1-OCP could not be induced into the osteoclasts under non-contact conditions (Fig. 3). For further verification, the 4T1 cells were inoculated into a central region of the R1-OCP cells and continued culturing for 4 days. It was found that only the R1-OCP cells in direct contact with the tumor cells were induced into the osteoclasts, and the R1-OCP cells outside the tumor cell range were not induced into the osteoclasts (Fig. 4). Therefore, a close contact with the tumor cell was a necessary spatial condition for the tumor-related osteoclast.

The above results indicated that there was a strict spatiotemporal coupling relationship between the tumor cell and the tumor-related osteoclast, particularly in terms of strict spatial colocalization. Since the targeting of the tumor cell was difficult, the acid-producing properties of the osteoclast made it an ideal target. Therefore, the local accurate killing of tumor bone metastasis lesion might be triggered by targeting the tumor-related osteoclast.

Based on this, a nanomaterial for physical killing targeting a tumor-osteoclast conjugate was designed.

### Embodiment 2 Synthesis of nanomaterial for physical killing targeting tumor-osteoclast conjugate

### 2.1 Tetracycline-modified sodium bicarbonate & disodium hydrogen phosphate nanoliposome

DSPE-PEG2000-NHS and tetracycline were dissolved in 10 mL of chloroform at a ratio of 20 mg:6.8 mg, 100 µL of triethylamine was added to regulate the pH to 8.4, a reaction was performed for 24 hours while stirring, the mixture was rotary evaporated and then dissolved in water, and excess tetracycline was removed using a dialysis method, so as to obtain a tetracycline-modified polyethylene glycol 2000 phospholipid. The tetracycline-modified polyethylene glycol 2000 phospholipid, lecithin, and cholesterol were dissolved in 10 mL of chloroform at a ratio of 20 mg:100 mg:16 mg and then rotary evaporated at 37 degrees Celsius using a rotary evaporation; 10 mL of a mixed solution of 60 mM sodium bicarbonate and 60 mM disodium hydrogen phosphate was added to perform ultrasonic hydration; the mixture was placed in a dialysis bag for dialysis; and extrusion granulation was performed to obtain the tetracycline-modified sodium bicarbonate & disodium hydrogen phosphate nanoliposome.

### 2.2 Alendronate sodium-modified sodium bicarbonate & disodium hydrogen phosphate nanoliposome

DSPE-PEG5000-NHS and alendronate sodium were dissolved in 10 mL of chloroform at a ratio of 20 mg:4.3 mg, 100 µL of triethylamine was added to regulate the pH to 8.4, a reaction was performed for 24 hours while stirring, the mixture was rotary evaporated and then dissolved in water, and excess alendronate sodium was removed using a dialysis method, so as to obtain an alendronate sodium-modified polyethylene glycol 5000 phospholipid. The alendronate sodium-modified polyethylene glycol 5000 phospholipid, lecithin, and cholesterol were dissolved in 10 mL of chloroform at a ratio of 30 mg:100 mg:16 mg and then rotary evaporated at 37 degrees Celsius using a rotary evaporation; 10 mL of a mixed solution of 30 mM sodium bicarbonate and 80 mM disodium hydrogen phosphate was added to perform ultrasonic hydration; the mixture was placed in a dialysis bag for dialysis; and extrusion granulation was performed to obtain the alendronate sodium-modified sodium bicarbonate & disodium hydrogen phosphate nanoliposome.

### 2.3 Tetracycline-modified sodium bicarbonate & disodium hydrogen phosphate nanoliposome

DSPE-PEG2000-NHS and tetracycline were dissolved in 10 mL of chloroform at a ratio of 20 mg:6.8 mg, 100 µL of triethylamine was added to regulate the pH to 8.0, a reaction was performed for 24 hours while stirring, the mixture was rotary evaporated and then dissolved in water, and excess tetracycline was removed using a dialysis method, so as to obtain a tetracycline-modified polyethylene glycol 2000 phospholipid. The tetracycline-modified polyethylene glycol 2000 phospholipid, lecithin, and cholesterol were dissolved in 10 mL of chloroform at a ratio of 45 mg:100 mg:16 mg and then rotary evaporated at 37 degrees Celsius using a rotary evaporation; 10 mL of a mixed solution of 50 mM sodium bicarbonate and 20 mM disodium hydrogen phosphate was added to perform ultrasonic hydration; the mixture was placed in a dialysis bag for dialysis; and extrusion granulation was performed to obtain the tetracycline-modified sodium bicarbonate & disodium hydrogen phosphate nanoliposome.

### 2.4 Tetracycline-modified sodium bicarbonate & disodium hydrogen phosphate nanoliposome

DSPE-PEG2000-NHS and tetracycline were dissolved in 10 mL of chloroform at a ratio of 20 mg:6.8 mg, 100 µL of triethylamine was added to regulate the pH to 8.2, a reaction was performed for 24 hours while stirring, the mixture was rotary evaporated and then dissolved in water, and excess tetracycline was removed using a dialysis method, so as to obtain a tetracycline-modified polyethylene glycol 2000 phospholipid. The tetracycline-modified polyethylene glycol 2000 phospholipid, lecithin, and cholesterol were dissolved in 10 mL of chloroform at a ratio of 30 mg:100 mg:16 mg and then rotary evaporated at 37 degrees Celsius using a rotary evaporation; 10 mL of a mixed solution of 120 mM sodium bicarbonate and 50 mM disodium hydrogen phosphate was added to perform ultrasonic hydration; the mixture was placed in a dialysis bag for dialysis; and extrusion granulation was performed to obtain the tetracycline-modified sodium bicarbonate & disodium hydrogen phosphate nanoliposome.

### Embodiment 3 Application example of tetracycline-modified sodium bicarbonate & disodium hydrogen phosphate nanoliposome for physically killing tumor-osteoclast conjugate in bone metastasis of breast cancer

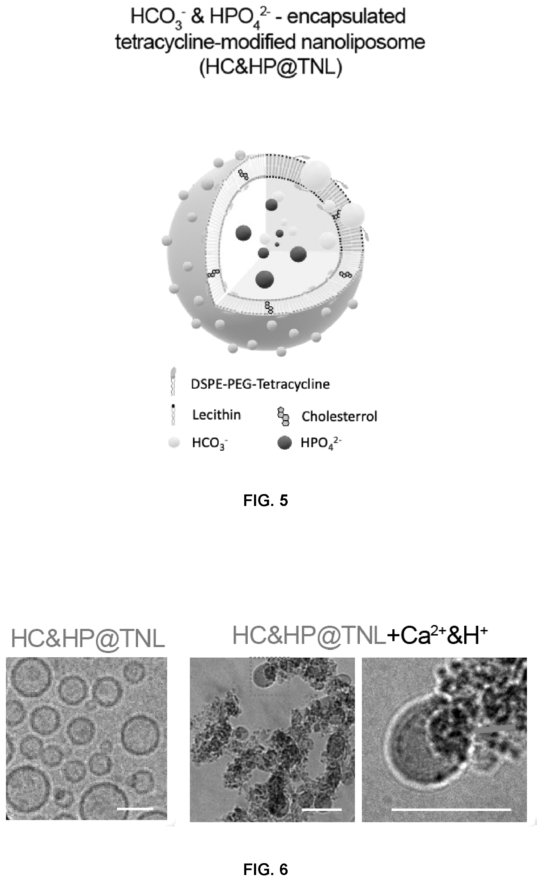

The tetracycline-modified sodium bicarbonate & disodium hydrogen phosphate nanoliposome prepared in Embodiment 2.1 with an average particle size being approximately 100 nanometers was selected, with a typical schematic structural diagram shown in Fig. 5, and the physicochemical properties of the nanoliposome to target a tumor-osteoclast conjugate were tested first.

### (1) Cryo-transmission electron microscopy:

1) A copper mesh was treated with glow discharge for 120 seconds to improve hydrophilicity, and 2.5 microliters of HC&HP@TNL (control or with a hydrochloric acid solution and a calcium chloride solution added to trigger its release) was added to the copper mesh.
2) Conditions were set to apply pressure for 5 s, then release the pressure for 3 s to form a thin liquid layer, and immerse it in liquid nitrogen for vitrification.
3) A sample was placed under a 200 kV cryo-transmission electron microscope for observation.
4) It was observed that liposomes, which were originally structurally intact, ruptured upon triggering, and released sodium hydrogen phosphate was combined with calcium ions to form a large number of crystals (Fig. 6).

### (2) Crystal element analysis:

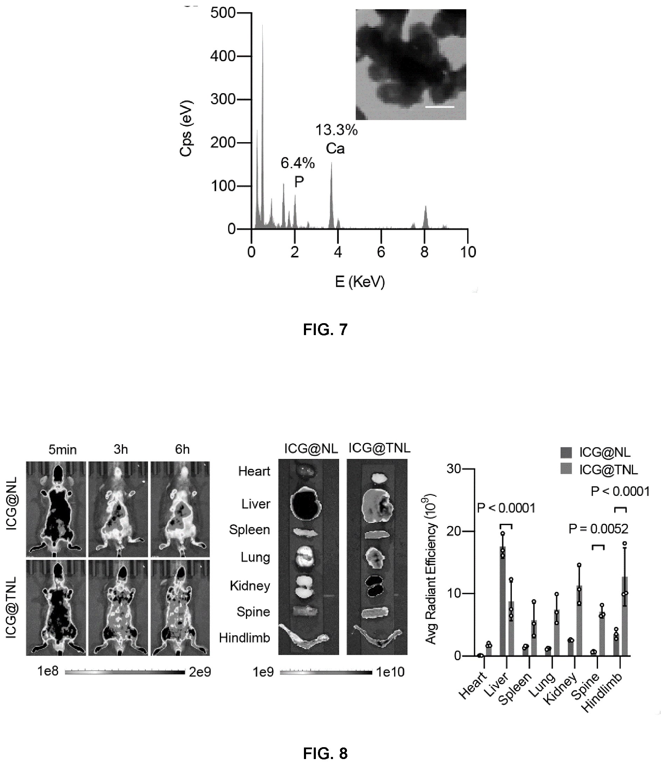

1) A copper mesh was treated with glow discharge for 120 seconds to improve hydrophilicity, and 2.5 microliters of HC&HP@TNL that was triggered by adding a hydrochloric acid solution and a calcium chloride solution was added to the copper mesh.
2) A sample was placed under a transmission electron microscope and subjected to EDS spectrum determination in a transmission mode, so as to determine the proportions of elements such as Ca, P, O, etc.
3) Through determination and calculation, it was verified that the crystals formed was calcium phosphate crystals (Fig. 7).

### (3) In vivo imaging and tracing:

1) 0.5 mg/mL of Indocyanine Green (ICG) was added during ultrasonic hydration of the HC&HP@TNL, so as to perform in vivo tracking.
2) The tetracycline-modified ICG@TNL and non-tetracycline-modified ICG@NL were injected into the Balb/c mice via tail vein at a dose of 10 mL/kg.
3) The distribution of liposomes in the mice was observed using IVIS in vivo imaging at 5 minutes, 3 hours, and 6 hours, respectively.
4) Through analysis of in vivo imaging data, it was found that the tetracycline-modified liposomes might effectively be enriched in bone tissue, with a bone-to-liver signal ratio of approximately 1 (Fig. 8).

### (4) Cell-specific release experiment:

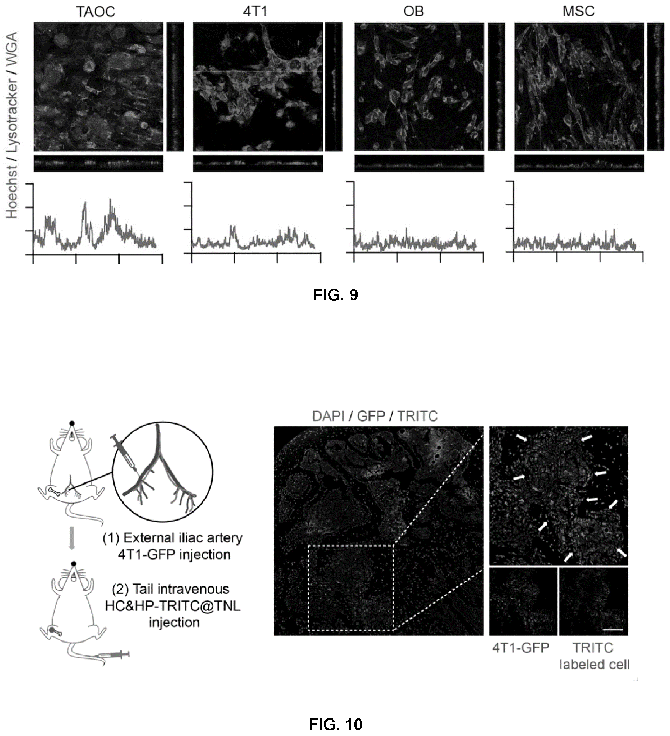

1) A pH probe Lysotracker was added during ultrasonic hydration of the HC&HP@TNL, so as to characterize liposome release.
2) A tumor-related osteoclast (TAOC), a 4T1 tumor cell, an osteoblast, and a mesenchymal stem cell were respectively cultured on bone fragments, and incubated in an incubator at 37 °C for 1 day using an α-MEM culture medium.
3) The liposomes were added to the culture medium at a volume ratio of 1:10, and cell membranes were stained and labeled using WGA-Rhod.
4) The bone fragments were placed upside down on a glass slide, and a confocal microscope was used to observe a Lysotracker release signal at an interface between the cells and the bone fragments.
5) Image data was analyzed, it was found that liposome release was observed only at a contact surface between the TAOC and the bone fragment, indicating that the TAOC might effectively trigger the release of the HC&HP@TNL for the killing of the tumor-osteoclast conjugate (Fig. 9).

### (4) In vivo release experiment:

1) 8-to-10-week-old female Balb/c mice were selected and anesthetized.
2) A 1.5-centimeter incision was formed in the lower right abdomen between the 4th and 5th nipples. The muscles were opened to expose the iliac artery.
3) 0.1 mL of 5 × 10⁶/mL GFP-expressing 4T1 breast tumor cells were injected into the iliac artery using a 31G needle.
4) The area of the arterial incision was pressed with a cotton-tipped swab to stop the bleeding, then the incision was sutured.
5) A TRITC fluorescent molecule was added during ultrasonic hydration of the HC&HP@TNL to achieve fluorescent positioning of a release region.
6) The liposome was injected into the mice via the tail vein at a dose of 10 mL/kg.
7) Tissue section was performed from mouse femurs, and fluorescence slice-scanning was performed.
8) A TRITC signal was detected at a tumor metastasis lesion, that is, there was a high degree of colocalization between the tumor cell and a range affected by liposome release, indicating that the HC&HP@TNL might achieve accurate release at the tumor metastasis lesion through triggering by the TAOC (Fig. 10).

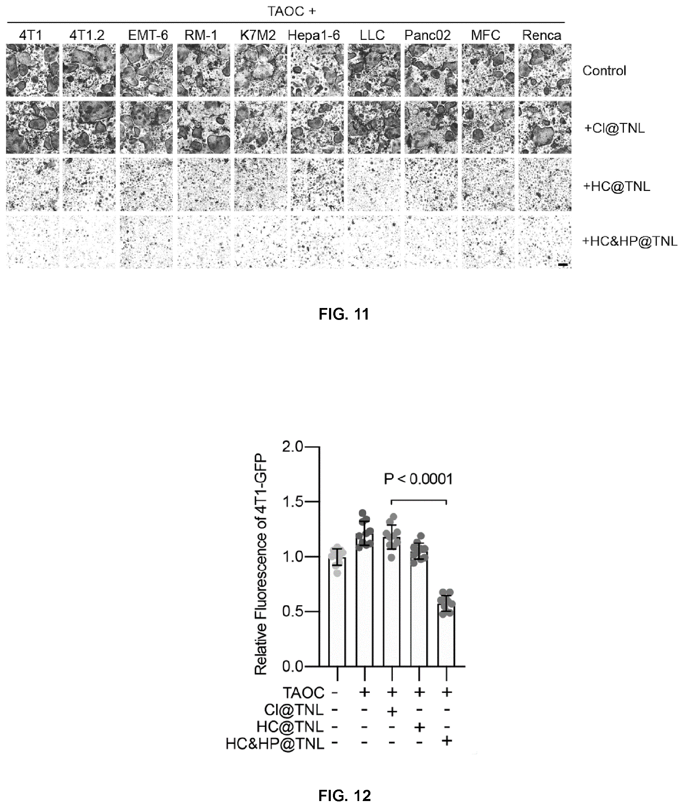

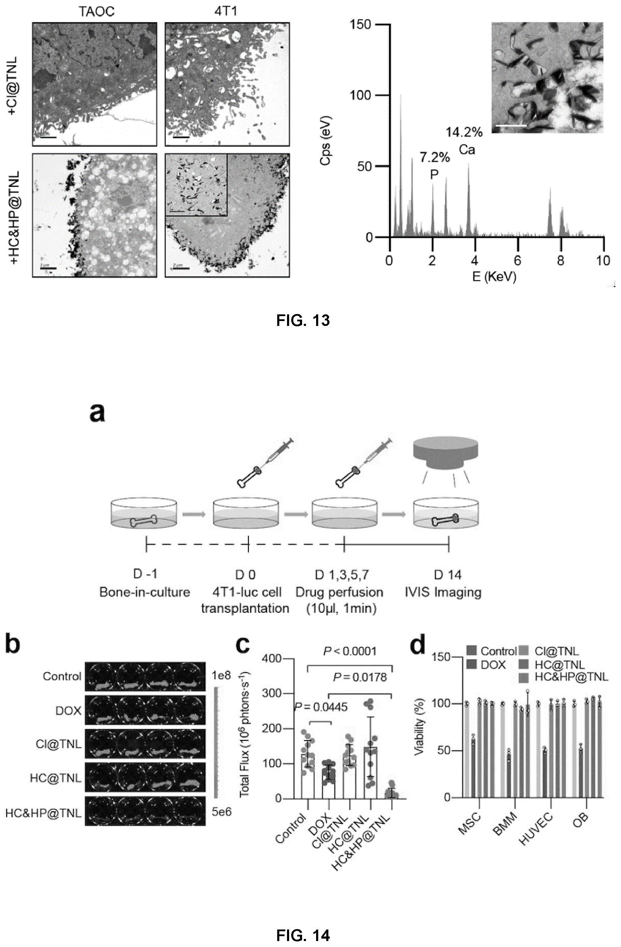

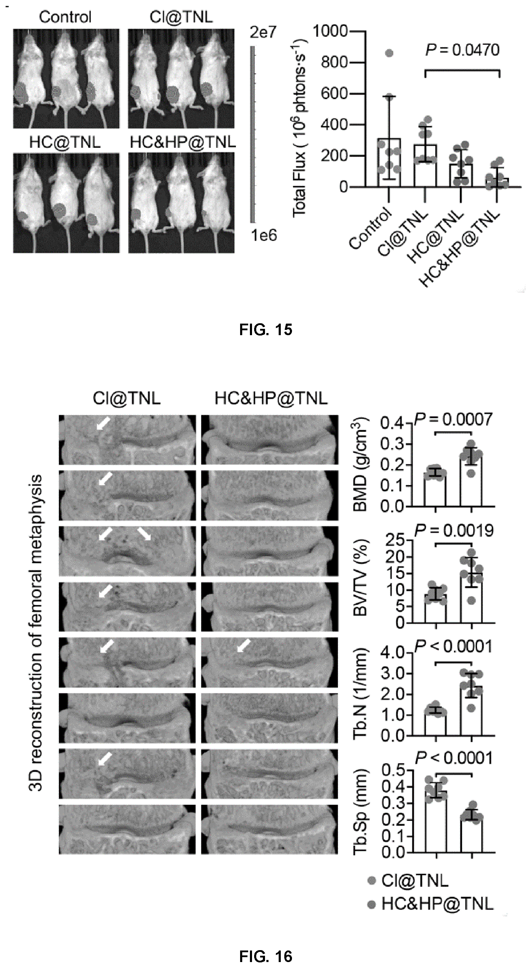

Then, the decoupling-killing effect of the HC&HP@TNL on the tumor-osteoclast conjugate was verified.
(1) Tumor-related osteoclast induction and decoupling-killing effect in tumors of different sources:
   1) Mouse femurs were taken and rinsed with a culture medium to obtain bone marrow cells, which were then cultured for 5 days in an α-MEM culture medium containing 20 ng/mL M-CSF.
   2) The cells were digested and spread onto a plate; Day 0 was set; 50 ng/mL RANKL was added to a culture system and incubated for 1 day, then the RANKL was removed to obtain R1-OCP; tumor cells from different sources (4T1, 4T1.2, EMT-6: breast cancer cells; RM-1: prostate cancer cells; K7M2: osteosarcoma cells; Hepa1-6: liver cancer cells; LLC: lung cancer cells; Panc02: pancreatic cancer cells; MFC: gastric cancer cells; and Renca: renal cancer cells) were added to the R1-OCP for co-culture.
   3) A treatment group was set, a tetracycline-modified nanoliposome encapsulating sodium chloride (Cl@TNL), a tetracycline-modified nanoliposome encapsulating sodium bicarbonate (HC@TNL), and the tetracycline-modified nanoliposome encapsulating sodium bicarbonate and disodium hydrogen phosphate (HC&HP@TNL) were respectively added to the culture medium at a volume ratio of 1:10.
   4) Continuous culture was performed until Day 5, then 4% paraformaldehyde was used for fixation, and TRAP staining was performed.
   5) It was found that a tumor-related osteoclast generation was a universal phenomenon observed across tumor cells of various sources. Furthermore, after the addition of the HC&HP@TNL, it was found that the HC&HP@TNL had a decoupling-killing effect on the tumor-osteoclast conjugates in various types of tumors (Fig. 11).
(2) Verification of tumor cell killing:
   1) The GFP-expressing 4T1 tumor cells were spread at a density of 1000/well into a 96-well cell culture plate of the R1-OCP cells with a density of 10000/well.
   2) The tetracycline-modified nanoliposome encapsulating sodium chloride (Cl@TNL), the tetracycline-modified nanoliposome encapsulating sodium bicarbonate (HC@TNL), and the tetracycline-modified nanoliposome encapsulating sodium bicarbonate and disodium hydrogen phosphate (HC&HP@TNL) respectively added to the culture medium at a volume ratio of 1:10.
   3) After 1 day, the 96-well cell culture plate was placed in a multifunctional microplate reader to quantify a GFP signal.
   4) The fluorescence intensity of GFP verified that the HC&HP@TNL caused killing and growth inhibition of the tumor cells under the triggering of the tumor-related osteoclast (Fig. 12).
   5) Glutaraldehyde fixation was performed on the killed cells, and transmission electron microscopy sample preparation was performed.
   6) After resin fixation, ultrathin sectioning was performed on the cell sample.
   7) Under the transmission electron microscopy, a large number of calcium phosphate crystals were observed on surfaces and inside both the TAOC and 4T1 cells, the integrity of the tumor cells was disrupted, thereby achieving direct physical killing to the tumor cells (Fig. 13).
(3) In-vitro evaluation of tumor inhibition effect:
   1) 8-to-10-week-old female Balb/c mice were selected, mouse femurs were separated, and the mouse femurs were cultured in vitro in a cell culture plate to construct a bone-in-culture array (BICA).
   2) On Day 0, Luciferase-expressing 4T1 tumor cells were injected into the femurs to construct a bone metastasis model.
   3) On Day 1, 3, 5, and 7, a microinjector was used to perfuse the tetracycline-modified nanoliposome encapsulating sodium chloride (Cl@TNL), the tetracycline-modified nanoliposome encapsulating sodium bicarbonate (HC@TNL), the tetracycline-modified nanoliposome encapsulating sodium bicarbonate and disodium hydrogen phosphate (HC&HP@TNL), and a classic anticancer drug DOX at a low perfusion rate of 10 microliters/minute into the femurs.
   4) On Day 14, 1.5 mg/mL of a potassium luciferin substrate was added to each sample, and results were compared through IVIS imaging (Fig. 14a).
   5) The effects of the HC&HP@TNL were compared with those of the traditional chemotherapeutic agent doxorubicin (DOX) in BICA; compared with the DOX, the HC&HP@TNL showed a more pronounced tumor inhibition effect and had fewer side effects in other cells (Fig. 14b-d).
(4) Mouse bone metastasis lesion inhibition experiment:
   1) 8-to-10-week-old female Balb/c mice were selected and anesthetized.
   2) A 1.5-centimeter incision was formed in the lower right abdomen between the 4th and 5th nipples. The muscles were opened to expose the iliac artery.
   3) 0.1 mL of 5 × 10⁶/mL GFP-expressing or Luciferase-expressing 4T1 breast tumor cells were injected into the iliac artery using a 31G needle.
   4) The area of the arterial incision was pressed with a cotton-tipped swab to stop the bleeding, then the incision was sutured, considered as Day 0.
   5) On Day 1, 3, 5, and 7, the tetracycline-modified nanoliposome encapsulating sodium chloride (Cl@TNL), the tetracycline-modified nanoliposome encapsulating sodium bicarbonate (HC@TNL), and the tetracycline-modified nanoliposome encapsulating sodium bicarbonate and disodium hydrogen phosphate (HC&HP@TNL) were respectively injected via the tail vein at a dose of 10 mL/kg.
   6) On Day 8, Micro-CT scanning was performed on the femurs of mice in each group that had been inoculated with the GFP-4T1 cells, and metaphyseal bone parameters were measured.
   7) On Day 28, 150 mg/kg of the luciferin potassium salt substrate was injected via the tail vein to the mice in each group that had been inoculated with the Luciferase-4T1 cells, and the growth of a tumor metastasis lesion was observed using IVIS imaging.
   8) Micro-CT and IVIS luminescence intensity data was analyzed, and it was found that the physical killing of the HC& HP@TNL to the tumor-osteoclast conjugate effectively suppressed bone metastasis lesions (Fig. 15) and significantly reduced bone loss at the metaphyseal region (BMD: 0.24 ± 0.04 g/cm³ vs. 0.17 ± 0.02 g/cm³, p = 0.0007; BV/TV: 15.44 ± 4.44% vs. 8.96 ± 1.81%, P < 0.0019; Tb.N: 2.43 ± 0.58 mm⁻¹ vs. 1.25 ± 0.14 mm⁻¹, P < 0.0001; Tb.Sp: 0.23 ± 0.03 mm vs. 0.38 ± 0.05 mm, P < 0.0001) (Fig. 16). This indicated that the nanomaterial for preventing tumor bone metastasis of the present invention might effectively inhibit a bone metastasis.

The above are merely preferred implementations of the present invention. It is to be noted that, although the present invention has been described in detail through the above preferred embodiments, those skilled in the art should understand that various improvements and modifications may be made without departing from the principles of the present invention, and such improvements and modifications should also be considered within the scope of protection of the present invention and do not deviate from the scope defined by the claims of the present invention.

## Claims

1. A nanomaterial, being a bone-targeting group-modified nanovesicle encapsulating a carbonate compound and a phosphate compound, wherein preferably, the nanovesicle comprises a nanoliposome.

2. The nanomaterial according to claim 1, wherein an average particle size of the nanomaterial is 50-1000 nanometers; preferably, the average particle size is 50-200 nanometers; and more preferably, the average particle size is below 100 nanometers.

3. The nanomaterial according to claim 1, wherein the bone-targeting group comprises at least one of tetracycline, phosphonate, calcein, or an aspartate peptide sequence; the phosphonate, for example, is alendronate sodium; preferably, the bone-targeting group is the tetracycline.

4. The nanomaterial according to claim 1, wherein the carbonate compound is selected from a carbonate salt or a bicarbonate salt, preferably selected from at least one of sodium bicarbonate, potassium bicarbonate, ammonium bicarbonate, sodium carbonate, potassium carbonate, or ammonium carbonate, and more preferably is sodium bicarbonate; the phosphate compound is selected from a hydrogen phosphate salt or a dihydrogen phosphate salt, preferably selected from at least one of disodium hydrogen phosphate, dipotassium hydrogen phosphate, diammonium hydrogen phosphate, sodium dihydrogen phosphate, potassium dihydrogen phosphate, or ammonium dihydrogen phosphate, and more preferably is disodium hydrogen phosphate; and preferably, a molar ratio of the carbonate compound to the phosphate compound is 1-4:4-1, preferably 1-3:3-1 or 1-2:2-1, and more preferably 1:1.

5. The nanomaterial according to claim 1, wherein a liposome membrane of the nanoliposome comprises a bone-targeted phospholipid, phospholipids other than the bone-targeted phospholipid, and cholesterol.

6. The nanomaterial according to claim 1, wherein the bone-targeted phospholipid is obtained by covalently bonding a functionalized pegylated phospholipid and a bone-targeting group molecule; preferably, the functionalized pegylated phospholipid refers to a pegylated phospholipid modified with a reactive functional group; the reactive functional group is hydroxyl, carboxyl, amino, or maleimide group; a molecular weight of polyethylene glycol is 500-50000, preferably 800-6000; more preferably, the functionalized pegylated phospholipid is functionalized DSPE-PEG; more preferably, the functionalized pegylated phospholipid is DSPE-PEG-NHS;
the phospholipids other than the bone-targeted phospholipid are at least one of natural phospholipids, semi-synthetic phospholipids, or fully-synthetic phospholipids; preferably, the phospholipids other than the bone-targeted phospholipid are at least one of lecithin, hydrogenated lecithin, or cephalin; more preferably, the phospholipids are lecithin; and
preferably, a mass ratio of the bone-targeted phospholipid, the phospholipids other than the bone-targeted phospholipid, and cholesterol is 5-40:100:10-25, preferably 15-25:100:12-20, and more preferably 20:100:16.

7. A method for preparing the nanomaterial according to any one of claims 1 to 6, comprising the following steps:
placing a bone-targeted phospholipid, phospholipids other than the bone-targeted phospholipid, and cholesterol in an organic solvent, removing the solvent, then adding a solution of a carbonate compound and a phosphate compound to perform ultrasonic hydration, placing the mixture in a dialysis bag for dialysis, and performing extrusion granulation to obtain a bone-targeting group-modified nanoliposome encapsulating the carbonate compound and the phosphate compound.

8. The method according to claim 7, wherein the bone-targeted phospholipid is prepared by the following method: covalently bonding a functionalized pegylated phospholipid and a bone-targeting group molecule, and obtaining the bone-targeted phospholipid through purification; preferably, reaction conditions for covalently bonding the functionalized pegylated phospholipid and the bone-targeting group molecule comprise: placing the functionalized pegylated phospholipid and the bone-targeting group molecule in an organic solvent, using an organic base to regulate the pH to 7.5-9.0, and performing, while stirring, a reaction for 6-48 hours at room temperature.

9. Use of the nanomaterial according to any one of claims 1 to 6 in preparation of drugs, wherein the drugs are used for killing a tumor-osteoclast conjugate in a bone metastasis lesion, or for preventing tumor bone metastasis.

10. The use according to claim 9, wherein the killing refers to physical killing; more preferably, the killing refers to physical killing caused by calcium phosphate crystals.

11. The use according to claim 9, wherein a bone metastasis lesion source comprises at least one of breast tumor, prostate tumor, bone tumor (comprising osteosarcoma), lung tumor, liver tumor, kidney tumor, gastrointestinal tumor (comprising gastric tumor and intestinal tumor), or pancreatic tumor; tumors in the tumor bone metastasis comprise at least one of breast tumor, prostate tumor, bone tumor (comprising osteosarcoma), lung tumor, liver tumor, kidney tumor, gastrointestinal tumor (comprising gastric tumor and intestinal tumor), or pancreatic tumor.

12. A method for culturing a tumor-related osteoclast, comprising co-culturing an osteoclast precursor pre-stimulated by RANKL and a tumor cell in a contact manner, so as to obtain a tumor-related osteoclast.

13. The culture method according to claim 12, wherein the osteoclast precursor pre-stimulated with the RANKL is an osteoclast precursor stimulated with the RANKL for 1-3 days, preferably the osteoclast precursor stimulated with the RANKL for 1 day; and tumors comprise at least one of breast tumor, prostate tumor, bone tumor (comprising osteosarcoma), lung tumor, liver tumor, kidney tumor, gastrointestinal tumor (comprising gastric tumor and intestinal tumor), or pancreatic tumor.
